# EUROPEAN PATENT APPLICATION

(11) **EP 4 835 809 A1**
(43) Date of publication of application: **04.11.2026**
(21) Application number: 25173839.9
(22) Date of filing: 01.05.2025
(51) Int. Cl.: G06T 7/00, A61B 8/00

(54) **PATHOLOGY DETECTION**

(71) Applicant: Koninklijke Philips N.V., 5656 AE Eindhoven (NL)
(72) Inventor: LI, Ye, Eindhoven (NL); CHEN, Alvin, Eindhoven (NL); CHEN, Li, 5656AG Eindhoven (NL)
(74) Representative: Philips Intellectual Property & Standards

(57) **Abstract**

Methods and systems for pathology detection and classification in ultrasound videos may analyze, using a first artificial intelligence (AI) model, individual ones of ultrasound images, wherein the first AI model outputs, for the individual ones, bounding box coordinates of a bounding box indicating a region where a pathology is detected. The system may apply a tracking algorithm to the bounding box coordinates to track the bounding box across the plurality of ultrasound images to generate a tracklet. The system may generate a video tubelet comprising features from regions of the plurality of ultrasound images included in the bounding box based on the tracklet. The system may analyze, using a second AI model, the video tubelet to generate a first determination of whether the video tubelet includes the pathology. A second determination may be made of whether the plurality of ultrasound images includes the pathology.

## Description

### FIELD OF THE INVENTION

The present disclosure relates to acquiring and analyzing medical imaging data. For example, embodiments herein relate to acquiring, and analyzing ultrasound imaging data using artificial intelligence (AI) model(s).

### BACKGROUND OF THE INVENTION

Ultrasound imaging is a widely used diagnostic tool for monitoring the health of fetuses, children, and adults. Artificial intelligence (AI) has been utilized to improve the diagnostic abilities of ultrasound, increasing its usefulness. However, AI models, which may use one or more algorithms, often require significant computational resources. The computational needs are typically met by the use of advanced hardware such as multiple graphical processing units, which may cost hundreds or thousands of dollars apiece. Rural and under resourced communities may lack the hardware needed to run AI algorithms that aid in ultrasound procedures. This scarcity of advanced hardware and technology may result in a considerable gap in healthcare for residents of these communities. Accordingly, algorithms that are less complex and may be run on lower tier hardware while maintaining adequate accuracy are desired.

### SUMMARY OF THE INVENTION

The invention is defined by the independent claims. The dependent claims define advantageous embodiments.

Embodiments of methods and systems for efficient pathology detection and classification in ultrasound videos on resource-constrained hardware may analyze, using a first artificial intelligence (AI) model, individual ones of ultrasound images, wherein the first AI model outputs, for the individual ones, bounding box coordinates of a bounding box indicating a region where a pathology is detected in. The system may apply a tracking algorithm to the bounding box coordinates to track the bounding box belonging to one detected object, across the plurality of ultrasound images, wherein the tracking algorithm generates a tracklet. The system may generate a video tubelet comprising features from regions of the plurality of ultrasound images included in the bounding box based on the tracklet. The system may analyze, using a second AI model, the video tubelet to generate a first determination of whether the video tubelet includes the pathology (e.g., one or more of the detected objects includes the pathology). The system may generate a second determination using the second AI model, based at least in part on the first determination, of whether the plurality of ultrasound images includes the pathology. The second determination is the maximum of all tubelet predictions in the ultrasound images (e.g., the ultrasound video). The ultrasound imaging system further comprises a display configured to provide the determination of the second AI model.

In accordance with at least one example disclosed herein, an ultrasound imaging system is disclosed. The ultrasound imaging system comprises at least one processor configured to analyze, using a first artificial intelligence (AI) model, individual ones of a plurality of ultrasound images, wherein the first AI model outputs, for the individual ones of the plurality of ultrasound images, bounding box coordinates of a bounding box indicating a region where a pathology is detected in for the individual ones of the plurality of ultrasound images. The at least one processor is further configured to apply a tracking algorithm to the bounding box coordinates to track the bounding box across the plurality of ultrasound images, wherein the tracking algorithm generates a tracklet. The at least one processor is further configured to generate a video tubelet comprising features from regions of the plurality of ultrasound images included in the bounding box based, at least in part, on the tracklet. The at least one processor is further configured to analyze, using a second AI model, the video tubelet to generate a first determination of whether the video tubelet includes the pathology. The at least one processor is further configured to generate a second determination using the second AI model, based at least in part on the first determination, of whether the plurality of ultrasound images includes the pathology. The ultrasound imaging system further comprises a display configured to provide the determination of the second AI model.

In some embodiments, the first AI model comprises a deep convolutional neural network, and wherein the first AI model is configured to localize candidate regions in the individual ones of the plurality of ultrasound images.

In some embodiments, the first AI model further outputs a class confidence of the pathology located in the bounding box.

In some embodiments, the video tubelet comprises a feature map generated by the first AI model of portions of the plurality of ultrasound images included in the bounding box.

In some embodiments, the at least one processor is further configured to embed spatial context information of the video tubelet using the second AI model. In some such embodiments, the spatial context information comprises at least one of an absolute location of the video tubelet in the plurality of ultrasound images and a relative location of the video tubelet in the plurality of ultrasound images, or a combination thereof.

In some embodiments, the second AI model comprises a spatiotemporal deep convolutional neural network classification model.

In some embodiments, the at least one processor is further configured to assign a class label to contents contained within the bounding box coordinates of the individual ones of the plurality of ultrasound images, based on a comparison of the bounding box coordinates, ground truth bounding box coordinates, and unified bounding box coordinates, wherein the unified bounding box coordinates correspond to a maximum area of all bounding box coordinates contained in the tracklet across the plurality of ultrasound images.

### BRIEF DESCRIPTION OF THE DRAWINGS

Fig. 1 illustrates a block diagram of an ultrasound imaging system arranged in accordance with principles of the present disclosure.
Fig. 2 illustrates a high-level block diagram describing embodiments discussed herein.
Fig. 3 illustrates an overall architecture describing embodiments discussed herein.
Fig. 4 illustrates an example schematic for pathology candidate detection, according to embodiments herein.
Fig. 5 illustrates a schematic for video tubelet formation, according to embodiments herein.
Fig. 6 illustrates an example of tubelet label assignment, according to embodiments herein.
Fig. 7 illustrates a schematic for video tubelet and video classification, according to embodiments herein.
Fig. 8 illustrates a method for detecting pathology in ultrasound imaging, according to embodiments herein.

### DETAILED DESCRIPTION OF EMBDIMENTS

The following description of certain examples is illustrative in nature and is in no way intended to limit the disclosed technology or its applications or uses. In the following detailed description of examples of the present systems, and methods, reference is made to the accompanying drawings, which form a part hereof, and in which are shown by way of illustration specific examples in which the described systems, and methods may be practiced. These examples are described in sufficient detail to enable those skilled in the art to practice the presently disclosed systems, and methods, and it is to be understood that other examples may be utilized, and that structural and logical changes can be made without departing from the spirit and scope of the present disclosure. Moreover, for the purpose of clarity, detailed descriptions of certain features will not be discussed when they would be apparent to those with skill in the art, so as not to obscure the description of the present technology. The following detailed description is therefore not to be taken in a limiting sense, and the scope of the present technology is defined only by the appended claims.

Complex algorithms such as large neural networks and other types of artificial intelligence (AI) models are becoming more common in medical imaging. For example, AI may be used for improved detection of anatomy and/or diagnosis. These advances may improve patient outcomes. However, users of medical imaging systems, such as ultrasound imaging systems, may not always have access to hardware for running these complex algorithms such as. Modern state-of-the-art hospitals and offices may have such hardware, however under-resourced communities may lack such access to hardware. As a result, under-resourced communities may not be able to run complex algorithms that various ultrasound procedures utilize, reducing the level of care that is provided in comparison to communities with state-of-the-art hardware.

Developing AI-based computer-aided detection (CAD) software for resource-constrained hardware requires balancing computational efficiency with representational learning capacity. Large/deeper AI models improve accuracy but increase computational demands, while small/shallow networks are efficient but may sacrifice accuracy. The challenge is to design the smallest/shallowest network that ensures accurate clinical diagnosis. For ultrasound video analysis, 2D detectors like YOLO perform well on individual frames, but cannot capture temporal patterns, limiting their diagnostic accuracy. In contrast, 3D detection models handle temporal data but are too computationally demanding for resource-constrained hardware.

Embodiments disclosed herein provide a lightweight AI framework for computer-aided pathology detection that analyzes complex spatiotemporal information contained in ultrasound videos. The AI framework may be computationally efficient enough to operate on resource-constrained hardware. Examples may include older ultrasound systems and compact ultrasound systems such as Philips Lumify, Philips Blaze/Flash, and Philips Compact. Instead of using a large 3D detection model, embodiments herein employ two lightweight AI models to analyze clinically relevant video sub-volumes, referred to as tubelets. This region-of-interest (ROI) approach focuses on critical spatial details and temporal patterns of the pathology, minimizing distractions from irrelevant areas while keeping computational demands low thus allowing for implementation on resource-constrained hardware.

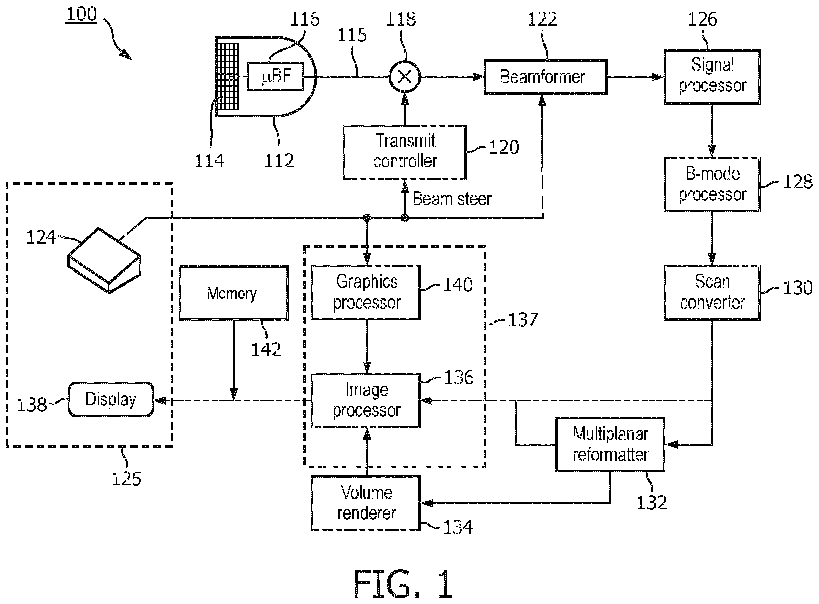

Fig. 1 illustrates a block diagram of an ultrasound imaging system 100 arranged in accordance with principles of the present disclosure. In the ultrasound imaging system 100 of Fig. 1, an ultrasound probe 112 includes a transducer array 114 for transmitting ultrasonic waves and receiving echo information. The transducer array 114 can be implemented as a linear array, convex array, a phased array, and/or a combination thereof. The transducer array 114, for example, can include a two-dimensional array (as shown) of transducer elements capable of scanning in both elevation and azimuth dimensions for 2D and/or 3D imaging. The transducer array 114 can be coupled to a microbeamformer 116 in the probe 112, which controls transmission and reception of signals by the transducer elements in the array. In this example, the microbeamformer 116 is coupled by the probe cable to a transmit/receive (T/R) switch 118, which switches between transmission and reception and protects the main beamformer 122 from high-energy transmit signals. In some embodiments, the T/R switch 118 and other elements in the system can be included in the ultrasound probe 112 rather than in a separate ultrasound system base. In some embodiments, the ultrasound probe 112 may be coupled to the ultrasound imaging system via a wireless connection (e.g., WiFi, Bluetooth).

The transmission of ultrasonic beams from the transducer array 114 under control of the microbeamformer 116 is directed by the transmit controller 120 coupled to the T/R switch 118 and the beamformer 122, which receives input from the user's operation of the user interface (e.g., control panel, touch screen, console) 125. The user interface 125 may include soft and/or hard controls. One of the functions controlled by the transmit controller 120 is the direction in which beams are steered. Beams may be steered straight ahead from (orthogonal to) the transducer array 114, or at different angles for a wider field of view. The partially beamformed signals produced by the microbeamformer 116 are coupled via channels 115 to a main beamformer 122 where partially beamformed signals from individual patches of transducer elements are combined into a fully beamformed signal. In some embodiments, microbeamformer 116 is omitted and the transducer array 114 is coupled via channels 115 to the beamformer 122. In some embodiments, the system 100 can be configured (e.g., include a sufficient number of channels 115 and have a T/R controller programmed to drive the transducer array 114) to acquire ultrasound data responsive to a plane wave or diverging beams of ultrasound transmitted toward the subject. In some embodiments, the number of channels 115 from the ultrasound probe may be less than the number of transducer elements of the transducer array 114 and the system can be operable to acquire ultrasound data packaged into a smaller number of channels than the number of transducer elements.

The beamformed signals are coupled to a signal processor 126. The signal processor 126 can process the received echo signals in various ways, such as bandpass filtering, decimation, I and Q component separation, and/or harmonic signal separation. The signal processor 126 can also perform additional signal enhancement such as speckle reduction, signal compounding, and noise elimination. The processed signals are coupled to a B-mode processor 128, which can employ amplitude detection for the imaging of structures in the body. The signals produced by the B-mode processor 128 are coupled to a scan converter 130 and a multiplanar reformatter 132. The scan converter 130 arranges the echo signals in the spatial relationship from which they were received in a desired image format. For instance, the scan converter 130 can arrange the echo signal into a two-dimensional (2D) sector-shaped format, or a pyramidal three-dimensional (3D) image. The multiplanar reformatter 132 can convert echoes, which are received from points in a common plane in a volumetric region of the body into an ultrasonic image of that plane, as described in U.S. Pat. No. 6,443,896 (Detmer).

A volume renderer 134 converts the echo signals of a 3D data set into a projected 3D image as viewed from a given reference point, e.g., as described in U.S. Pat. No. 6,530,885 (Entrekin et al.). In some implementations, the system 100 can additionally or alternatively be configured to perform 3D and/or 4D acquisitions, such as protocolized 3D/4D acquisitions. The 2D or 3D images can be coupled from the scan converter 130, multiplanar reformatter 132, and volume renderer 134 to at least one processor 137 for further image processing operations. For example, the at least one processor 137 can include an image processor 136 configured to perform further enhancement and/or buffering and temporary storage of imaging data for display on an image display 138. However, in some embodiments, the display 138 may not display ultrasound images to the user.

The display 138 can include a display device implemented using a variety of display technologies, such as LCD, LED, OLED, or plasma display technology. The at least one processor 137 can include a graphics processor 140, which can generate graphic overlays for display with the ultrasound images. These graphic overlays can contain, e.g., standard identifying information such as patient name, date and time of the image, imaging parameters, and the like. For these purposes the graphics processor 140 receives input from the user interface 125, such as a typed patient name. The user interface 125 can also be coupled to the multiplanar reformatter 132 for selection and control of a display of multiple multiplanar reformatted (MPR) images.
The user interface 125 can include one or more mechanical controls, such as buttons, dials, a trackball, a physical keyboard, and others, which may also be referred to herein as hard controls. Alternatively or additionally, the user interface 125 can include one or more soft controls, such as buttons, menus, soft keyboard, and other user interface control elements implemented for example using touch-sensitive technology (e.g., resistive, capacitive, or optical touch screens). One or more of the user controls can be co-located on a control panel 124. For example, one or more of the mechanical controls can be provided on a console and/or one or more soft controls can be co-located on a touch screen, which can be attached to or integral with the console. The display 138 and the user interface 125 can be included in an I/O component, via which outputs are provided by the system 100 and/or inputs are received by the system 100.

In some implementations, the user interface 125 can receive inputs and provide outputs of the disclosed system. For example, the user interface 125 can receive a user input specifying a desired pathology, and/or patient information. In some implementations the one or more outputs of the system can be provided via one or more graphical user interfaces (e.g., via the display 138).

The at least one processor 137 (e.g., the image processor 136, the graphics processor 140, or a different processor) can perform functions associated with acquiring and configuring medical imaging data, as described herein. For example, the at least one processor 137 may implement one or more algorithms, including one or more AI models. According to embodiments of the present disclosure, the at least one processor 137 may analyze, using a first AI model, ultrasound images generated by the ultrasound imaging system 100. For example, the images may have been generated in part by image processor 136. The first AI model may output bounding box coordinates of a bounding box indicating a region where a pathology is detected in for the ultrasound images. The pathology may be an anatomy of interest (e.g., fetal heart, carotid artery) or a lesion (e.g., tumor, regions of liver sclerosis). The processor 137 may apply a tracking algorithm to generate temporally consistent bounding boxes for each object detected anatomy or pathology of interest across all the ultrasound images contained in the video. This may allow for tracking temporal patterns. The tracking algorithm generates a tracklet. The processor 137 may utilize the tracklet to generate a video tubelet including a sub-volume of the ultrasound video included in the bounding box tracked over time by the tracklet. The processor 137 may implement a second AI model to analyze the video tubelet to generate a first determination of whether the video tubelet includes the pathology or anatomy of interest. A second determination may be generated based on the final video-level confidence, which is the maximum of all video tubelet predictions in the video (e.g., the ultrasound images). In some cases, the final video-level classification (e.g., whether the video includes the pathology) is determined by the combined predictions of the first and second AI models in some cases. The determination may be provided to the user interface 125, for example, display 138. In some examples, the image processor 136 may implement the AI models and tracking algorithm.

Although described as separate processors, it will be understood that the functionality of any of the processors described herein can be implemented in a single processor (e.g., a CPU or graphical processing units (GPU) implementing the functionality of processor 137) or fewer number of processors than described in this example. In some embodiments, the at least one processor 137 can be hardware-based (e.g., include multiple layers of interconnected nodes implemented in hardware). In some embodiments, the at least one processor 137 can be implemented at other processing stages, e.g., prior to the processing performed by the image processor 136, volume renderer 134, multiplanar reformatter 132, and/or scan converter 130. In some embodiments, the at least one processor 137 can be implemented to process ultrasound data in the channel domain, beamspace domain (e.g., before or after beamformer 122), the in-phase and quadrature phase (IQ) domain (e.g., before, after, or in conjunction with signal processor 126), and/or the k-space domain. As described, in some embodiments, functionality of two or more of the processing components (e.g., beamformer 122, signal processor 126, B-mode processor 128, scan converter 130, multiplanar reformatter 132, volume renderer 134, at least one processor 137, image processor 136, graphics processor 140, etc.) can be combined into a single processing unit and/or divided between multiple processing units. The processing units can be implemented in software, hardware, or a combination thereof. For example, the at least one processor 137 can include one or more GPU(s). In another example, beamformer 122 can include an application specific integrated circuit (ASIC).

The at least one processor 137 can be coupled to one or more computer-readable media (e.g., memory 142) included in the system 100, which can be non-transitory. The one or more computer-readable media can carry instructions and/or a computer program that, when executed, cause the at least one processor 137 to perform operations described herein. For example, the memory 142 may include instructions for implementing the AI models and/or tracking algorithm. A computer program can be stored/distributed on any suitable medium, such as an optical storage medium or a solid-state medium supplied together with or as part of other hardware, and can also be distributed in other forms, such as via the Internet or other wired or wireless telecommunication systems. Furthermore, embodiments can take the form of a computer program product accessible from a computer-readable medium providing program code for use by or in connection with a computer or any device or system that executes instructions. For the purposes of this disclosure, a computer-readable medium can generally be any tangible apparatus that can contain, store, communicate, propagate, and/or transport the program for use by or in connection with the instruction execution device. The computer-readable medium can be, for example, without limitation, an electronic, magnetic, optical, electromagnetic, infrared, or semiconductor system, and/or a propagation medium. Non-limiting examples of a computer readable medium include a semiconductor or solid-state memory, magnetic tape, a removable computer diskette, a random-access memory (RAM), a read-only memory (ROM), a rigid magnetic disk, and/or an optical disk. Optical disks can include compact disk read only memory (CD-ROM), compact disk-read/write (CD-R/W), and/or DVD.

In some embodiments, while shown separately, many of the components of system 100 may be included within the probe 112. In some embodiments, some of the components of system 100 may be included in a tablet or other portable computing device. In some applications, this may provide an ultrasound imaging system that may be more portable and/or more suitable for care in remote/rural environments compared to traditional cart-based ultrasound systems. An example of such a portable system is the Philips^{®} Lumify^{®} system. However, this is merely an example, and the disclosure is not limited to this specific system.

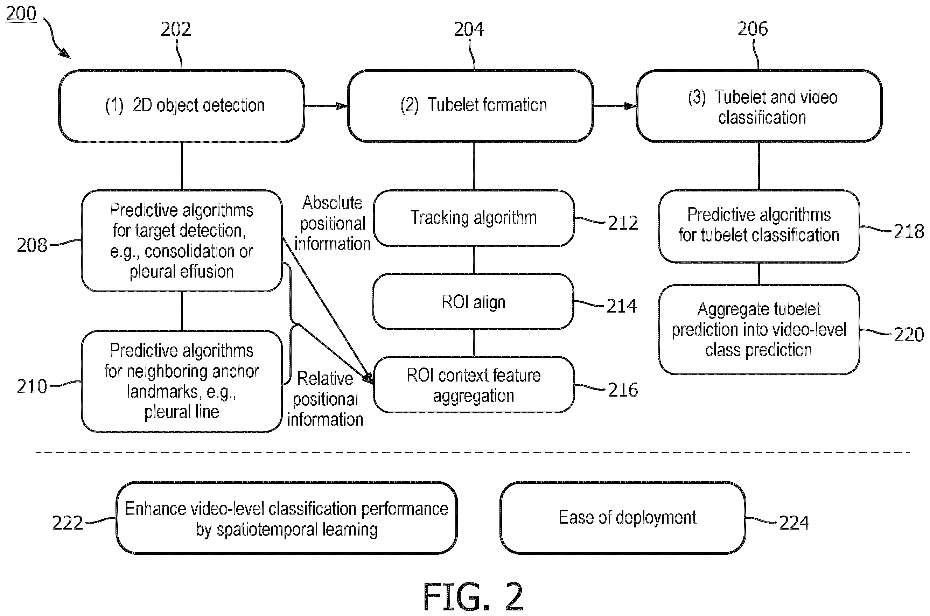

Fig. 2 illustrates a high-level block diagram 200 describing embodiments discussed herein.

Embodiments herein for pathology detection and classification include three stages as illustrated in block diagram 200. Note that the block diagram 200 may be performed by the system 100 of Fig. 1.

A first stage includes performing 2D object detection 202 on single images of a performed ultrasound procedure. In some cases, predictive algorithms 208 may be used for target detection (e.g., consolidation or pleural effusion) to predict absolute positional information of the target in the form of a bounding box (e.g., coordinates), within which a pathology is most likely to reside. Further, other predictive algorithms 210 may be used for determining where neighboring anchor landmarks reside (e.g., pleural line) to provide auxiliary information to the second stage (e.g., performing tubelet formation). Note that the predictive algorithms 208 may also be used to determine positional information of a bounding box relative to its neighboring anchor landmarks. The absolute positional information and relative positional information may be passed to the second stage of the block diagram 200.

A second stage includes performing tubelet formation 204 based on the 2D object detection 202. For example, to construct the tubelet a tracking algorithm 212 may be applied to the output of the 2D object detection 202 stage (e.g., predicted bounding boxes on a frame-level). Further, a ROI alignment (ROI align) algorithm 214 (further discussed herein) may be applied to the output of the tracking algorithm 212. Additionally, a ROI context feature aggregation 216 (further discussed herein) may be performed on the scalar outputs of the predictive algorithms 208 and the predictive algorithms 210 (e.g., based on the absolute positional information and relative positional information received from the 2D object detection 202).

A third stage includes tubelet classification 206, where a tubelet is classified as either containing a pathology or not containing a pathology. For example, predictive algorithms 218 may be used for tubelet classification (e.g., having or not having the pathology). Further, tubelet prediction aggregation 220 may be performed to aggregate the tubelet predictions belonging to the same video into the video-level class prediction of that video for output to the user.

Embodiments herein may enhance 222 video-level classification performance by using spatiotemporal learning. For example, embodiments herein may improve pathology detection and classification as, unlike CAD systems that use static 2D detector for frame-level object detection and video classification of an entire video, embodiments herein first identify potential regions of pathology (e.g., using bounding boxes) and re-exam these regions (e.g., using video tubelets) across multiple frames. By leveraging multi-frame data within the video tubelets, embodiments herein process temporal patterns which may allow more accurate classification at much lower computational costs compared to 3D detector models that process the whole video.

Embodiments herein may also improve computational efficiency and/or enhance 224 ease of deployment. For example, with 0.4M parameters, the tubelet-based video pathology classification model discussed herein integrates into detection-based CAD systems (such as YOLO), which may improve ease of deployment.

Additionally, embodiments herein introduce global context in the tubelet classification 206 stage. For example, the tubelet location, size, and detection confidence are embedded as additional inputs in the tubelet classification 206 stage. Such embedding of contextual information preserves global spatial context for the tubelet after ROI alignment.

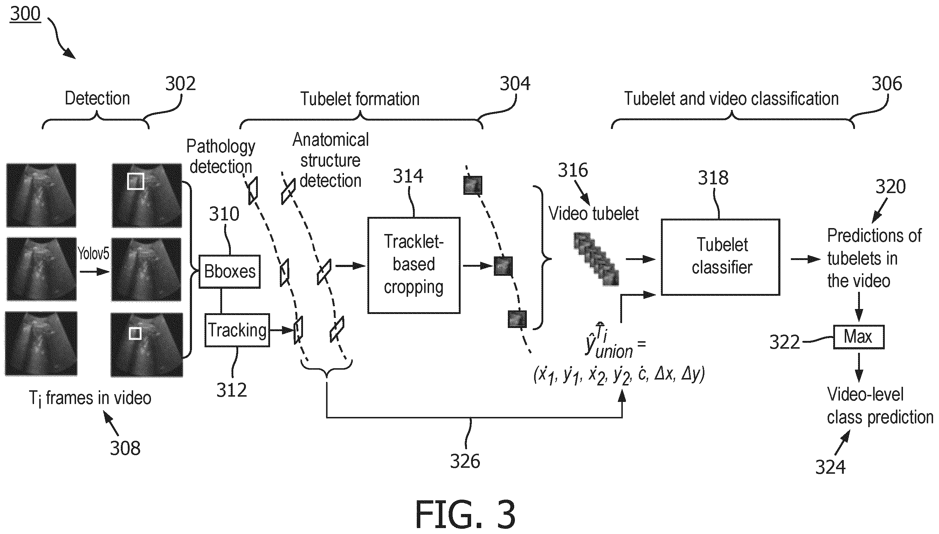

Fig. 3 illustrates an overall architecture 300 describing embodiments discussed herein.

The architecture 300 of Fig. 3 may be performed by the system 100 described in Fig. 1.

In various embodiments, to extract the spatial and temporal features about the underlying pathology, a 2D detector may be used to predict the spatial features on each frame of a video and the 2D detector may connect the predicted spatial features (as given by a bounding box) temporally via a tracking algorithm. Accordingly, the temporally refined bounding boxes (e.g., tracklets) are used to crop the original video to generate 3D candidate sub-volume(s) within each video (e.g., tubelets). A lightweight second-stage classification network is trained to focus on examining the 3D (e.g., 2D and time) candidate sub-volumes (e.g., tubelets), minimizing the disruption from the complex and variable background which occupies a large fraction of the input volume.

For example, the 2D detector may perform pathology candidate detection 302. A deep convolutional neural network (CNN) (e.g., a 2D object detection model) may be used to localize candidate regions of pathology within each ultrasound image frame 308 of an ultrasound video. The deep convolutional neural network may take one frame (e.g., 2D ultrasound image frame 308) of an ultrasound video and may output predicted bounding box coordinates 310 and a class confidence of detected candidate pathology regions within the input ultrasound image frame 308. The deep CNN may output predicted bounding box coordinates 310 and a class confidence of detected candidate pathology regions for all of the ultrasound image frames 308 included in the ultrasound video in some embodiments.

Accordingly, video tubelet formation 304 may be performed (e.g., 2D and time aggregation) by applying a tracking algorithm 312 to connect predicted bounding box coordinates 310 to obtain temporally consistent/refined bounding box tracklets. Video tubelets 316 are constructed using the tracklets by performing tracklet-based cropping 314 (further discussed herein). Global spatial contextual information (e.g., where the tubelet resides in the whole ultrasound image) may be embedded into each video tubelet 316 (e.g., absolute location of the video tubelet 316 within the image and/or relative location of the video tubelet 316 to anatomical landmarks). Additionally, unified coordinates 326 ( ${\hat{y}}_{union}^{{\hat{T}}_{i}}$) may be calculated (further discussed herein) based on the tracklets obtained form the tracking algorithm 312 and may be used in the tubelet and video classification 306 as an input in the tubelet classifier 318.

In various embodiments, the tubelet and video classification 306 are performed based on the formed video tubelets 316. For example, a lightweight spatiotemporal classification model (e.g., a tubelet classifier 318) may classify the tubelets as either positive or negative for pathology (e.g., positive may mean the pathology is present and negative may mean the pathology is not present in the sub-volume). In some embodiments, the tubelet classifier 318 functions in a feature map space. In some examples, the tubelet classifier 318 may output tubelet classification predictions for several tubelets in the ultrasound video. In some examples, the final video-level confidence is the maximum 322 of all tubelet predictions in the video, thus generating a video-level class prediction that may be outputted 324 to the user (e.g., via display 138). In some cases, the lightweight spatiotemporal classification model may be a deep convolutional neural network, hardware permitting.

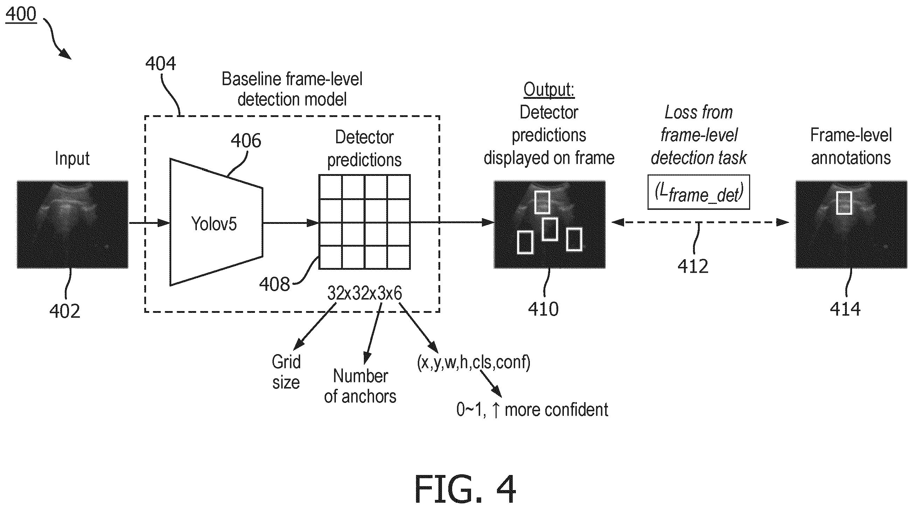

Fig. 4 illustrates an example schematic 400 for pathology candidate detection, according to embodiments herein.

The schematic 400 for pathology candidate detection further describes the 2D object detection 202 of the block diagram 200 of Fig. 2 and further describes the detection 302 of the architecture 300 of Fig. 3. The schematic 400 may be performed by the system 100 of Fig. 1.

In various embodiments, a detection model is trained with frame-level annotations to perform pathology candidate region detection. The detection model may be understood as a frame-level localization AI model. In some instances, the model is a deep learning network (hardware permitting). The AI model processes N frames from an ultrasound video clip and generates bounding boxes (e.g., bounding box coordinates) that identify the predicted regions/areas of target pathology in each frame. The architecture of the AI model can either be pre-existing or newly introduced and trained. Training of this frame-level localization AI model may utilize supervised learning with frame-level annotation labels. Specifically, each image frame used during training is paired with a corresponding ground-truth annotation (bounding boxes for detection tasks or free-form masks for segmentation tasks).

For example, as illustrated in schematic 400, a frame-level detection model 404 may be used for predicting candidate regions of target pathology. The frame-level detection model 404 takes an ultrasound image frame 402 of an ultrasound video as an input. The frame-level detection model 404 uses a YOLO architecture 406 to generate detection candidates 408 in the form of bounding boxes, which essentially are six-dimensional vectors including elements such as an x coordinate of the center of the bounding box, a y coordinate of the center of the bounding box, a width of the bounding box, a height of the bounding box, the predicted class index of the detected object in the bounding box (e.g., 0 for "consolidation" and 1 for "pleural effusion"), and a confidence score that a detected object is indeed present in the bounding box (e.g., value range of 0 to 1). Note that a plurality of detection candidates may be generated for each frame (i.e., in standard network designs, as many as 32*32*3 detections (bounding boxes) may be generated per frame). The frame-level detection model 404 may output the detection candidates 408 displayed on frame 410 (e.g., predicted boxes). The detection candidates 408 displayed on frame 410 are compared with ground truth annotation(s) on the frame to determine loss 412 from the frame-level detection. Accordingly, the predictions are regularized in shape (e.g., IoU-based loss and L1/L2 losses), regularized in classification class (e.g., cross-entropy loss) and regularized in confidence e.g., (binary cross-entropy loss). The regularized prediction (e.g., predicted bounding box coordinates) is then passed to the tubelet formation 204/ tubelet formation 304 stage of pathology detection. While frame 410 is shown in Fig. 4 to illustrate the output of the frame-level detection model 404, in some embodiments, frame 410 is not provided on a display of the ultrasound imaging system.

In some cases, the regularized prediction is outputted to the user (e.g., to the sonographer on display 138) as frame level annotations 414 on the ultrasound image frame with the predicted bounding box coordinates illustrated on the ultrasound image frame. In such cases, the predicted bounding box coordinates are used to generate the bounding boxes on the display. However, in other embodiments, the frame level annotations 414 may be used as input for another algorithm or model and not provided on the display.

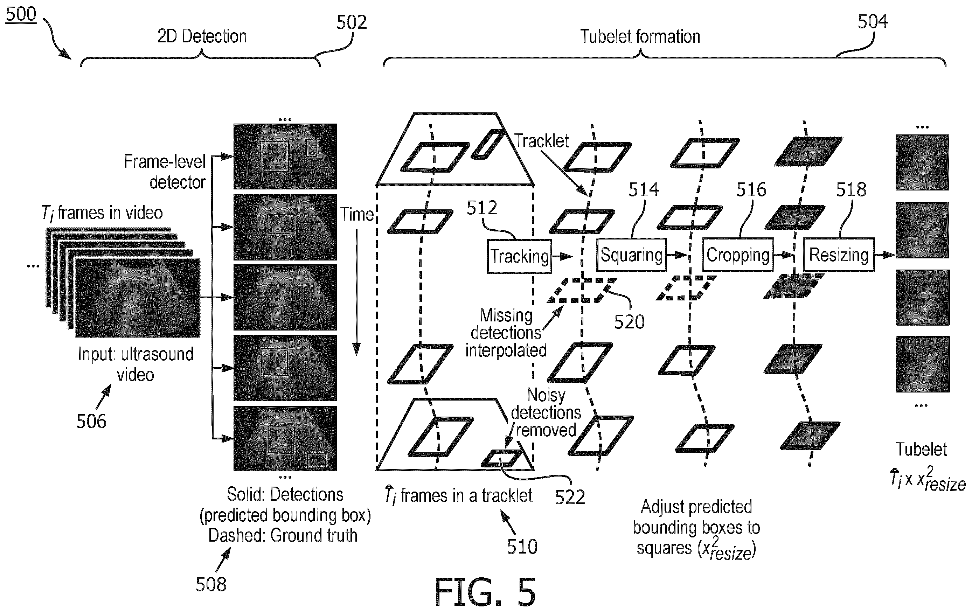

Fig. 5 illustrates a schematic 500 for video tubelet formation, according to embodiments herein.

The schematic 500 for video tubelet formation further describes the tubelet formation 204 of the block diagram 200 of Fig. 2 and further describes the tubelet formation 304 of the architecture 300 of Fig. 3. The schematic 500 may be performed by the system 100 of Fig. 1.

In various embodiments, instead of relying on frame prediction aggregation methods to produce a video-level classification, a feature-based approach may be used integrating visual/2D spatial and temporal information into video tubelets. Further, embodiments herein introduce a set of auxiliary non-image inputs to help classify the video tubelets. The tubelet-based approach may allow the AI framework to take temporal changes in the video into account while still providing real-time display of detection results during live scan. By real-time display, it means the ultrasound system provides a frame rate of 10 or more frames per second. Tubelets are processed through a lightweight spatiotemporal model, with tubelet location, size, and detection confidence embedded as additional inputs to provide global context.

To arrive at representations of temporally consistent regions of pathology along the video sequence, tracklets 510 are generated from each video in the frame-level labeled data and video-level labeled data (if available) by applying object tracking 512 directly on bounding box coordinate predictions from the 2D object detection 502 discussed herein. The bounding box coordinate predictions 508 are generated by the 2D object detection 502 as ultrasound video frames 506 are inputted into a model. The output of the model used in the 2D object detection 502 are bounding box coordinate predictions 508. Note that the 2D object detection 502 is further discussed herein in Fig. 4.

The bounding box predictions 508 are passed to an algorithm for tubelet formation 504. In some instances, a simple and efficient SORT tracker may be used as the tracking 512 algorithm for the tracklets 510, however note that other tracking techniques may be used. If any noisy bounding box predictions 508 are provided to the tubelet formation 504, the noisy bounding box predictions 508 are removed 522 from the tracklet 510. In some examples, a predicted bounding box may be considered "noisy" if the pathology of interest is not predicted in its neighboring frames in the ultrasound video (e.g., 3-5 frames). If bounding box predictions 508 are missing in the collection of tracklets at a certain time, a bounding box prediction may be interpolated 520 for that certain time based on the bounding box predictions 508 before the certain time and after the certain time.

The predicted bounding boxes coordinates within each tracklet are represented as {*ŷ¹,...,ŷ^{t}*}*,* where t ∈ (1, *T̂*ᵢ) is the number of frames of a tracklet. Each box vector y contains coordinates (x̂₁,ŷ₁,x̂₂,ŷ₂) and class confidence c . To fully preserve the native temporal changes in the detection ROI, for each tracklet a set of unified coordinates ${\hat{y}}_{union}^{{\hat{T}}_{i}} = \left({\dot{x}}_{1}, {\dot{y}}_{1}, {\dot{x}}_{2}, {\dot{y}}_{2}, \dot{c}\right)$ are calculated covering the maximum area of all bounding boxes, which may enhance the temporal consistency and field of view for each instance. The unified coordinates are squared 514 (e.g., rescaled to a square area) with its equal height and width which is equal to the maximum of the width and height from the unified coordinates. This may be understood as adjusting the predicted bounding box coordinates to squares (e.g., ${x}_{resize}^{2}$)*.* For each tracklet, the rescaled union coordinates ${\hat{y}}_{union}^{{\hat{T}}_{i}}$ are used to extract the video tubelet from original input ultrasound video. The video tubelets may be understood as a cropping 516 of each of the original ultrasound image frames to the size of the tracklet. Accordingly, the tubelet images A are resized 518 to *x_{resize}* × *x_{resize} × Tᵢ.*

In some cases, instead of cropping on the original video to form video tubelets, embodiments herein introduce the use of an ROI alignment algorithm, ROIAlign, to aggregate detector features into tubelets. The tubelet formation technique may allow a significant speed improvement in the pipeline and a performance gain by reusing feature representations learned through the strongly supervised detection loss. For each tracklet, the union coordinates ${\hat{y}}_{union}^{{\hat{T}}_{i}}$ are used as an input to the RolAlign layer to extract the video tubelet *fᵢ*: ${F}_{k} = concat \left({F}_{k}^{1},…,{F}_{k}^{T},axis=T\right) ∈ {R}^{C \times h \times w \times T}$ (1) ${f}_{i} = ROIAlign \left({F}_{k}, {\hat{y}}_{union}^{{\hat{T}}_{i}}, {x}_{resize}\right)$ (2) where ${F}_{k}^{i} ∈ {R}^{C \times h \times w \times T}$ is the k^{th} convolutional layer's feature maps across all video frames. The variables C, h, and w denote feature map channels, height, and width per frame, respectively. The output dimension after ROIAlign is denoted by x_{resize}.

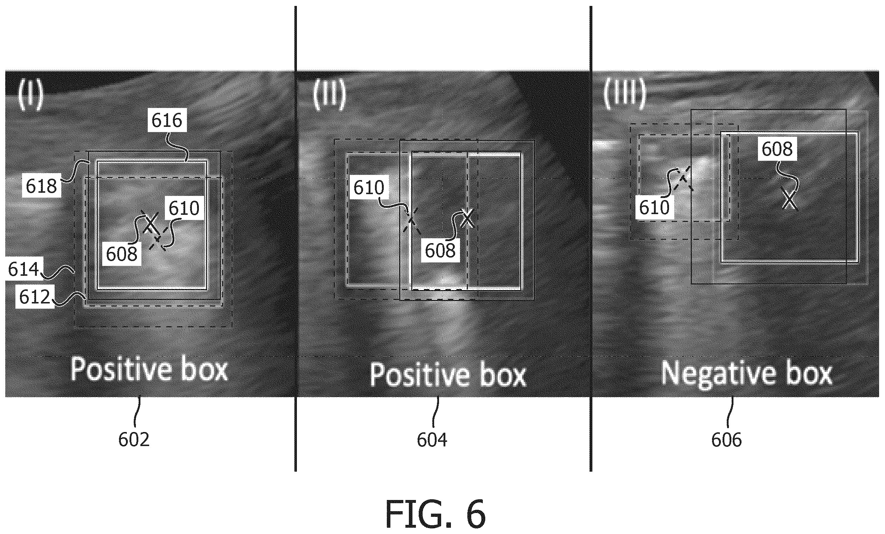

Fig. 6 illustrates an example of tubelet label assignment, according to embodiments herein.

The tubelet label assignment is performed as part of the block diagram 200 of Fig. 2 and the architecture 300 of Fig. 3 by the system 100 of Fig. 1.

In various embodiments, one or more labels may be assigned to each formed tubelet (e.g., the tubelets formed as described in Fig. 5), in both the instance- and tubelet-level. For example, the instance/per-frame class label for each ROI frame in a tubelet, *Lₜ* (where t ∈ (1, *T̂*ᵢ)), is determined by its relationship with the frame-level ground truth boxes. Given a frame-level bounding box label *y^{t}* = $\left({x}_{1}^{t}, {y}_{1}^{t}, {x}_{2}^{t}, {y}_{2}^{t}\right)$ and the unified predicted box ${\hat{y}}_{union}^{{\hat{T}}_{i}} = \left({\dot{x}}_{1}, {\dot{y}}_{1}, {\dot{x}}_{2}, {\dot{y}}_{2}, \dot{c}\right)$, for the same frame, a positive class label ${L}_{i}^{t} = 1$ is assigned if the center of the unified box and any ground truth box in that frame fall within each other's extents based on equations (3) : $\left\{\begin{matrix}{x}_{1}^{t} \leq \frac{{\dot{x}}_{1} + {\dot{x}}_{2}}{2} \leq {x}_{2}^{t} , {y}_{1}^{t} \leq \frac{{\dot{y}}_{1} + {\dot{y}}_{2}}{2} \leq {y}_{2}^{t} \\ {\dot{x}}_{1} \leq \frac{{x}_{1}^{t} + {x}_{2}^{t}}{2} \leq {\dot{x}}_{2} , {\dot{y}}_{1} \leq \frac{{y}_{1}^{t} + {y}_{2}^{t}}{2} \leq {\dot{y}}_{2}\end{matrix}\right.$ (3)

At the tubelet-level, a label is assigned to each tubelet by aggregation of all the instance labels based on equation (4): ${y}_{i} = \left\{\begin{matrix}1 , if {\sum }_{t = 1}^{{\hat{T}}_{i}} {L}_{i}^{t} > 0 , \\ 0 , otherwise\end{matrix}\right.$ (4)

For example, a positive case 602 (e.g., value of 1) may be when the center of the predicted tracklet bounding box falls within the extends of the ground truth bounding box in that frame. A borderline positive case 604 (e.g., value of 1) may be where the center of the predicted tracklet bounding box falls nears the edge of the ground truth bounding box. A negative case 606 (e.g., value of 0) may be when the center of the unified tracklet bounding box falls outside of the ground truth bounding box. The outer dashed box 614 and outer solid box 618 represent the to-be-cropped regions from the predicted and ground truth bounding box, respectively. The inner dashed box 612 and the inner solid box 616 represent the predicted and ground truth bounding boxes, respectively. The dashed crosshair mark 610 and the solid crosshair mark 608 represents the center of the predicted and ground truth bounding box, respectively.

It should be understood that all of the tubelets generated from videos may be kept in the frame-level labeled data, since reliable tubelet-level and instance-level labels can be derived from the existing ground truth frame-level bounding box labels. However, true labels from tubelets generated from the video-level labeled data are not known (since they could have come from true or false positive detections). Thus, in some cases, from the video-level labeled data, only tracklets with sufficient prediction confidences are used to construct tubelets and used to train the tubelet classifier model. Note that in some embodiments, the tubelet classifier functions in the feature space, thus making the classifier more efficient for hardware-constrained implementations.

In various embodiments, to address the loss of global context during tubelet classification, additional non-image inputs to the video tubelet classifier may be embedded in the video tubelet. In some instances, global context information may be inputted to the video tubelet classifier as a second input auxiliary to the main (e.g., tubelet) input. For example, the unified video tubelet's location, size, and detection confidence from the detector, ${\hat{y}}_{union}^{{\hat{T}}_{i}}$ may be inputted to the video tubelet classifier (e.g., tubelet classifier 318) as additional inputs. This allows the video tubelet classifier to gain global context about the video tubelet that it is looking at (e.g., where the video tubelet is located within the larger ultrasound video). In addition or alternatively to the absolute positional information about the video tubelet in the whole image, the relative location of the video tubelet may be inputted to the video tubelet classifier, as additional inputs. The relative location of the video tubelet can be calculated by first identifying an anatomical structure from the video. The relative position, Δ*x* and Δ*y* can be derived by calculating the distance from ${\hat{y}}_{union}^{{\hat{T}}_{i}}$*.* For example, for consolidation and pleural effusion detection in lung ultrasound videos, it may be that the relative positional information helps to confirm and exclude consolidation and pleural effusion predictions, in addition to the absolute global context information and the image features present in the tubelet.

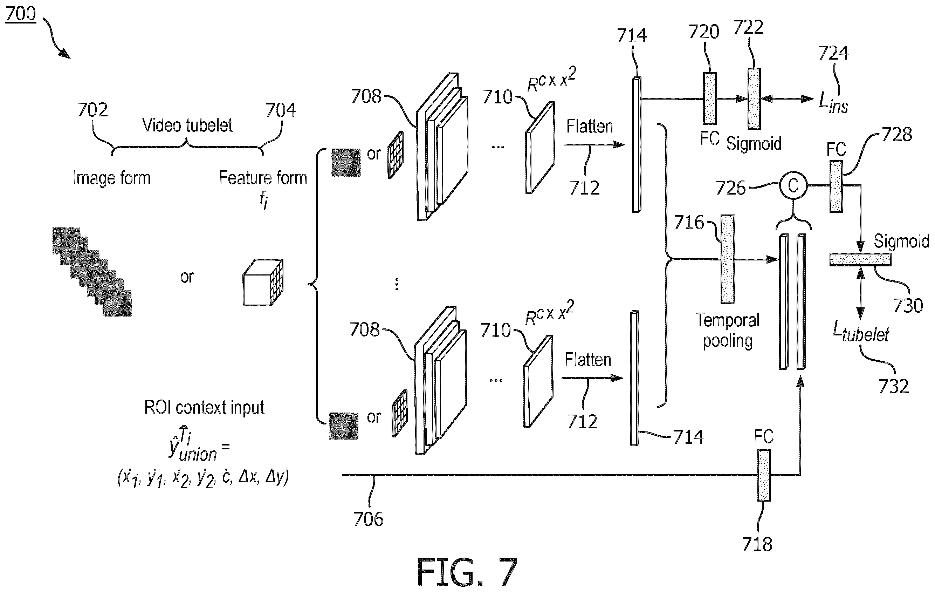

Fig. 7 illustrates a schematic 700 for video tubelet and video classification, according to embodiments herein.

The schematic 700 for video tubelet and video classification further describes the tubelet classification 206 of the block diagram 200 of Fig. 2 and further describes the tubelet and video classification 306 of the architecture 300 of Fig. 3. The schematic 700 may be performed by the system 100 of Fig. 1.

In various embodiments, the schematic 700 may receive the formed video tubelet from the tubelet formation 504 of schematic 500. Note that the video tubelet may be received in an image form 702 that may need to be further processed into a feature form 704 (e.g., a feature map), or be received in a feature form 704 (e.g., a feature map). The feature form 704 (e.g., feature map) may be received from the tubelet formation 504 described in Fig. 5 before the cropping 516 step where only feature information based on the predicted bounding box coordinates is provided and no ultrasound image is cropped to fit the feature information. Additionally, ROI contextual information 706 (e.g., ${\hat{y}}_{union}^{{\hat{T}}_{i}}$) may be received from schematic 500 for video tubelet formation 504.

In various embodiments, the introduced tubelet classifier processes the entire video tubelet by first encoding each instance in each frame and temporally pooling the encoded instance-level features to form the tubelet-level feature, which is used for tubelet-level classification. For example, each instance ( ${R}^{C \times {x}_{resize}^{2}}$) is an RoIAligned feature extracted from the detector's feature maps based on predicted bounding boxes within a unified tracklet. These are passed through a shared CNN encoder 708 (e.g., with N convolutional blocks) to obtain encoded instance-level image features 710 (*R^{C×x²}*)*.* The features are flattened 712 and sent to a shared fully connected layer 714 for instance-level classification, supervised by the instance level class label ${L}_{i}^{t} = 1$ as described in Fig. 6. Temporal pooling 716 is applied using a max-pooling layer across all flattened instance features in the tubelet, forming a one-dimensional (1-D) tubelet feature. This 1-D tubelet image feature is concatenated 726 with the embedded non-image tubelet feature (e.g., obtained by embedding the contextual information 706 through a fully connected layer 718), and the combined feature is fed into another fully connected layer 728 and a sigmoid function 730 for tubelet-level classification, supervised by *yᵢ* obtained using equation 4 discussed herein. The output of the sigmoid function 730 are predicted tubelet probabilities 732 (e.g., L_{tubelet}). The final predicted probability for video-level classification is the maximum of all tubelet probabilities 732 (e.g., max of L_{tubelet}) in the video. At the video-level, this may be understood as a binary output: either a pathology has been detected, or a pathology has not been detected.

In some instances, the flattened 712 features (flattened *R^{C×x²})* sent to the shared fully connected layer 714 may be further sent to a fully connected layer 720 and a sigmoid function 722 to obtain instance level predictions 724 (e.g., Lᵢₙₛ) of the video tubelet.

In some cases, tubelet-level classification probabilities 732 are provided (e.g., displayed on display 138) to the user (e.g., sonographer) in combination with the ultrasound image frame and the predicted bounding box coordinates of where the pathology is predicted to be. Additionally, the confidence of the prediction may be provided to the user. If the ultrasound scan is completed and no pathologies are detected, the user may be notified of the negative result and/or notified to redo the scan.

It should be understood that the AI model of schematic 700 is generating two determinations according to embodiments herein. The first determination made is whether the video tubelet includes the pathology. The second determination made is whether the ultrasound images (e.g., a video) include the pathology. Note that the second determination is generated, in part, based on the first determination. For example, in some embodiments, the second determination may be based, at least in part, on the maximum of all tubelet predictions.

Note that the procedure described herein is repeated for each ultrasound image frame of the ultrasound video thus outputting an artificial intelligence (AI) annotated video of each ultrasound image frame with the predicted bounding box coordinates and the binary output (e.g., a pathology has been detected or not). This AI annotated video may be generated and provided according to embodiments herein in near-real time.

While embodiments herein generally refer to ultrasound imaging and detection and classification of pathologies in ultrasound imaging, embodiments discussed herein may also be used for non-ultrasound-based imaging and detecting and classifying non-ultrasound features in videos when only resource-constrained hardware is available. Further, while examples provided herein describe analyzing videos on the imaging system, in other examples, the embodiments disclosed herein may be performed on a separate computing device (e.g., the videos of images are exported from the imaging device to the computing device).

Additionally, embodiments herein are often referred to as being implemented on resource-constrained hardware. However, it should be understood that embodiments herein may be implemented using complex hardware with ample resources and computing power.

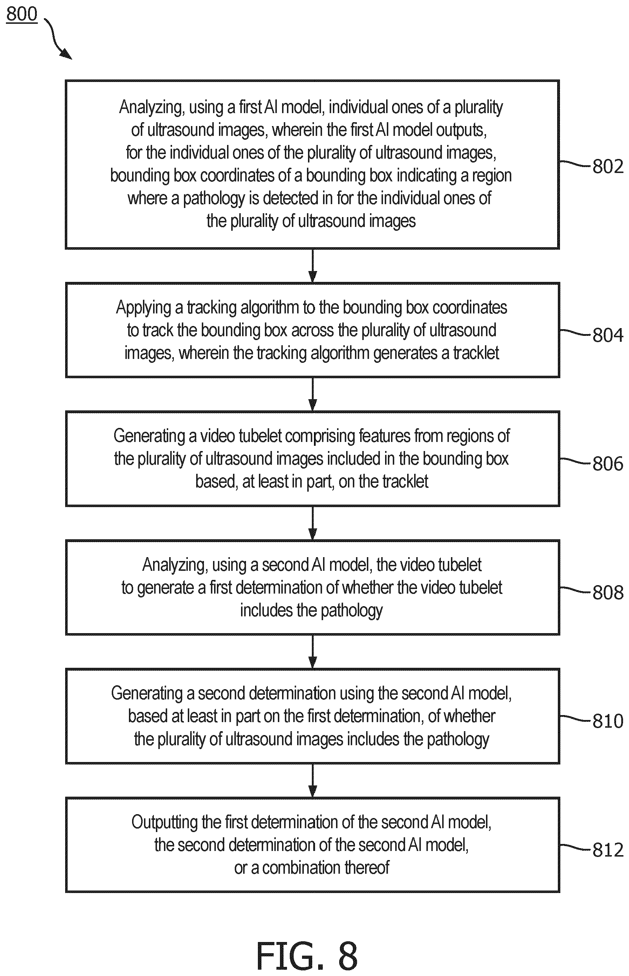

Fig. 8 illustrates a method 800 for detecting pathology in ultrasound imaging, according to embodiments herein.

The illustrated method 800 includes analyzing 802, using a first AI model, individual ones of a plurality of ultrasound images, wherein the first AI model outputs, for the individual ones of the plurality of ultrasound images, bounding box coordinates of a bounding box indicating a region where a pathology is detected in for the individual ones of the plurality of ultrasound images. For example, in 2D object detection 202 of block diagram 200, in pathology candidate detection 302 of architecture 300, and schematic 400 individual ones of ultrasound images are analyzed by an AI model to output bounding box coordinates.

The method 800 further includes applying 804 a tracking algorithm to the bounding box coordinates to track the bounding box across the plurality of ultrasound images, wherein the tracking algorithm generates a tracklet. For example, in schematic 500 tracklets 510 are generated.

The method 800 further includes generating 806 a video tubelet comprising features from regions of the plurality of ultrasound images included in the bounding box based, at least in part, on the tracklet. For example, in video tubelet formation 204 of block diagram 200, in tubelet formation 304 of architecture 300, and in video tubelet formation 504 of schematic 500 video tubelets are generated.

The method 800 further includes analyzing 808, using a second AI model, the video tubelet to generate a first determination of whether the video tubelet includes the pathology. For example, in tubelet classification 206 of block diagram 200, tubelet and video classification 306 of architecture 300, and schematic 700 a second AI model analyzes the video tubelet to determine whether the video tubelet includes a pathology or not.

The method 800 further includes generating 810 a second determination using the second AI model, based at least in part on the first determination, of whether the plurality of ultrasound images includes the pathology. For example, in tubelet classification 206 of block diagram 200, tubelet and video classification 306 of architecture 300, and schematic 700 a second AI model analyzes the video tubelet to determine whether the ultrasound image includes a pathology or not based on the first determination.

The method 800 further includes outputting 812 the first determination of the second AI model, the second determination of the second AI model, or a combination thereof. For example, the determination of the second AI model may be outputted onto display 138 of system 100.

In some embodiments of the method 800, the first AI model comprises a deep convolutional neural network, and wherein the first AI model is configured to localize candidate regions in the individual ones of the plurality of ultrasound images. For example, in schematic 400 the frame-level detection model 404 localizes candidate regions in the individual ones of the plurality of ultrasound images.

In some embodiments of the method 800, the first AI model further outputs a class confidence of the pathology located in the bounding box. For example, in schematic 400 the frame-level detection model 404 outputs a class confidence of the pathology located in the bounding box.

In some embodiments of the method 800, the video tubelet comprises a feature map generated by the first AI model of portions of the plurality of ultrasound images included in the bounding box. For example, in schematic 500 in tubelet formation 504 the video tubelet comprises a feature map that may be used as an input for the second AI model.

In some embodiments, the method 800 further comprises embedding spatial context information of the video tubelet using the second AI model. In some such embodiments, the spatial context information comprises at least one of an absolute location of the video tubelet in the plurality of ultrasound images, a relative location of the video tubelet in the plurality of ultrasound images, or a combination thereof. For example, in schematic 500 the video tubelet, during tubelet formation 504, may have spatial context predicted by the first AI model.

In some embodiments of the method 800, the second AI model comprises a spatiotemporal deep convolutional neural network classification model. For example, in schematic 700, a shared CNN encoder 708 (e.g., second AI model) is used to obtain encoded instance-level image features 710.

The systems and methods disclosed herein detect and classify pathology in ultrasound videos on resource-constrained hardware. Under resourced and underserved communities often do not have access to hardware that is needed to run complex algorithms for analyzing and parsing through ultrasound scan data. Thus, the quality of care in under resourced communities may be lower as compared to communities with ample access to state-of-the-art hardware. As such, embodiments herein introduce methods for pathology detection and classification that may be performed on hardware that is readily available in under resourced communities thus increasing the level of care provided to the under resourced community.

In various examples where components, systems and/or methods are implemented using a programmable device, such as a computer-based system or programmable logic, it should be appreciated that the above-described systems and methods can be implemented using any of various known or later developed programming languages, such as "Python", "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure, it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general-purpose processing circuits which are programmed responsive to executable instructions to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and methods may be that conventional medical image systems can be easily upgraded to incorporate the features and advantages of the present systems, devices, and methods.

Of course, it is to be appreciated that any one of the examples, examples or processes described herein may be combined with one or more other examples, examples and/or processes or be separated and/or performed amongst separate devices or device portions in accordance with the present systems, devices and methods.

Finally, the above-discussion is intended to be merely illustrative of the present systems and methods and should not be construed as limiting the appended claims to any particular example or group of examples. Thus, while the present system has been described in particular detail with reference to exemplary examples, it should also be appreciated that numerous modifications and alternative examples may be devised by those having ordinary skill in the art without departing from the broader and intended scope of the present systems and methods as set forth in the claims that follow. Accordingly, the specification and drawings are to be regarded in an illustrative manner and are not intended to limit the scope of the appended claims.
Example 1. An ultrasound imaging system comprising:
   at least one processor configured to:
   analyze, using a first artificial intelligence (AI) model, individual ones of a plurality of ultrasound images, wherein the first AI model outputs, for the individual ones of the plurality of ultrasound images, bounding box coordinates of a bounding box indicating a region where a pathology is detected in for the individual ones of the plurality of ultrasound images;
   apply a tracking algorithm to the bounding box coordinates to track the bounding box across the plurality of ultrasound images, wherein the tracking algorithm generates a tracklet;
   generate a video tubelet comprising features from regions of the plurality of ultrasound images included in the bounding box based, at least in part, on the tracklet;
      analyze, using a second AI model, the video tubelet to generate a first determination of whether the video tubelet includes the pathology; and
   generate a second determination, based at least in part on the first determination, of whether the plurality of ultrasound images includes the pathology; and
   a display configured to provide the first determination of the second AI model, the second determination of the second AI model, or a combination thereof.
Example 2. The ultrasound imaging system of Example 1, wherein the first AI model comprises a deep convolutional neural network, and wherein the first AI model is configured to localize candidate regions in the individual ones of the plurality of ultrasound images.
Example 3. The ultrasound imaging system of Example 1, wherein the first AI model further outputs a class confidence of the pathology located in the bounding box.
Example 4. The ultrasound imaging system of Example 1, wherein the video tubelet comprises a feature map generated by the first AI model of portions of the plurality of ultrasound images included in the bounding box.
Example 5. The ultrasound imaging system of Example 1, wherein the at least one processor is further configured to embed spatial context information of the video tubelet using the second AI model.
Example 6. The ultrasound imaging system of Example 5, wherein the spatial context information comprises at least one of an absolute location of the video tubelet in the plurality of ultrasound images and a relative location of the video tubelet in the plurality of ultrasound images, or a combination thereof.
Example 7. The ultrasound imaging system of Example 1, wherein the second AI model comprises a spatiotemporal deep convolutional neural network classification model.
Example 8. The ultrasound imaging system of Example 1, wherein the at least one processor is further configured to assign a class label to contents contained within the bounding box coordinates of the individual ones of the plurality of ultrasound images, based on a comparison of the bounding box coordinates, ground truth bounding box coordinates, and unified bounding box coordinates, wherein the unified bounding box coordinates correspond to a maximum area of all bounding box coordinates contained in the tracklet across the plurality of ultrasound images.
Example 9. A computer-implemented method for detecting pathology in ultrasound imaging, the method comprising:
   analyzing, using a first artificial intelligence (AI) model, individual ones of a plurality of ultrasound images, wherein the first AI model outputs, for the individual ones of the plurality of ultrasound images, bounding box coordinates of a bounding box indicating a region where a pathology is detected in for the individual ones of the plurality of ultrasound images;
   applying a tracking algorithm to the bounding box coordinates to track the bounding box across the plurality of ultrasound images, wherein the tracking algorithm generates a tracklet;
   generating a video tubelet comprising features from regions of the plurality of ultrasound images included in the bounding box based, at least in part, on the tracklet;
   analyzing, using a second AI model, the video tubelet to generate a first determination of whether the video tubelet includes the pathology;
   generating a second determination, based at least in part on the first determination, of whether the plurality of ultrasound images includes the pathology; and
   outputting the first determination of the second AI model, the second determination of the second AI model, or a combination thereof.
Example 10. The computer-implemented method of Example 9, wherein the first AI model comprises a deep convolutional neural network, and wherein the first AI model is configured to localize candidate regions in the individual ones of the plurality of ultrasound images.
Example 11. The computer-implemented method of Example 9, wherein the first AI model further outputs a class confidence of the pathology located in the bounding box.
Example 12. The computer-implemented method of Example 9, wherein the video tubelet comprises a feature map generated by the first AI model of portions of the plurality of ultrasound images included in the bounding box.
Example 13. The computer-implemented method of Example 9, further comprising embedding spatial context information in the video tubelet using the second AI model.
Example 14. The computer-implemented method of Example 13, wherein the spatial context information comprises at least one of an absolute location of the video tubelet in the plurality of ultrasound images, a relative location of the video tubelet in the plurality of ultrasound images, or a combination thereof.
Example 15. The computer-implemented method of Example 9, wherein the second AI model comprises a spatiotemporal deep convolutional neural network classification model.
Example 16. At least one non-transitory computer-readable medium carrying instructions that, when executed by at least one processor of an ultrasound imaging system, cause the ultrasound imaging system to:
   analyze, using a first artificial intelligence (AI) model, individual ones of a plurality of ultrasound images, wherein the first AI model outputs, for the individual ones of the plurality of ultrasound images, bounding box coordinates of a bounding box indicating a region where a pathology is detected in for the individual ones of the plurality of ultrasound images;
   apply a tracking algorithm to the bounding box coordinates to track the bounding box across the plurality of ultrasound images, wherein the tracking algorithm generates a tracklet;
   generate a video tubelet comprising features from regions of the plurality of ultrasound images included in the bounding box based, at least in part, on the tracklet; and
   analyze, using a second AI model, the video tubelet to generate a first determination of whether the video tubelet includes the pathology;
      generate a second determination, based at least in part on the first determination, of whether the plurality of ultrasound images includes the pathology; and
   output the first determination of the second AI model, the second determination of the second AI model, or a combination thereof.
Example 17. The non-transitory computer-readable medium of Example 16, wherein the first AI model comprises a deep convolutional neural network, and wherein the first AI model is configured to localize candidate regions in the individual ones of the plurality of ultrasound images.
Example 18. The non-transitory computer-readable medium of Example 16, wherein the first AI model further outputs a class confidence of the pathology located in the bounding box.
Example 19. The non-transitory computer-readable medium of claim 16, wherein the video tubelet comprises a feature map generated by the first AI model of portions of the plurality of ultrasound images included in the bounding box.
Example 20. The non-transitory computer-readable medium of Example 16, wherein the second AI model comprises a spatiotemporal deep convolutional neural network classification model.

## Claims

1. A computer-implemented method for image processing, the method comprising:
analyzing a plurality of ultrasound images to:
- identify in the plurality of ultrasound images a candidate region of interest of a pathology of interest, and
- generate for the plurality of ultrasound images a bounding box comprising the pathology of interest;
connecting the generated bounding boxes to generate a tracklet;
generating a video tubelet from the plurality of ultrasound images based at least in part on the tracklet;
analyzing the video tubelet to generate a determination of whether the tubelet comprises the pathology of interest.

2. The method of claim 1, wherein the plurality of ultrasound images is analyzed with a first AI

3. The method of claim 2, wherein the first AI model comprises a deep convolutional neural network.

4. The method of claim 3, wherein the first AI model further outputs a class confidence of the pathology located in the bounding box.

5. The method of any of the preceding claims, wherein the method further comprises:
- analyzing a plurality of ultrasound images to generate for the plurality of ultrasound images a feature map; and
wherein generating the video tubelet is further based on the generated feature maps.

6. The method of any one of the preceding claims, further comprising:
- embedding spatial context information in the video tubelet.

7. The method of any one of the preceding claims, wherein the spatial context information comprises at least one or a combination of:
- an absolute location of the video tubelet in the plurality of ultrasound images,
- a relative location of the video tubelet in the plurality of ultrasound images.

8. The method of any one of the preceding claims, wherein the video tubelet is analyzed with a second AI model.

9. The method of claim 8, wherein the second AI model comprises a spatiotemporal deep convolutional neural network classification model.

10. The method of any one of the preceding claims, further comprising:
- assigning a class label to contents contained within the bounding box of the plurality of ultrasound images, based on a comparison of
- the bounding box,
- a ground truth bounding box, and
- a unified bounding box,
wherein the unified bounding box corresponds to a maximum area of all bounding boxes contained in the tracklet across the plurality of ultrasound images.

11. The method of any one of the preceding claims, wherein the tracklet comprises a temporally consistent bounding box.

12. The method of any one of the preceding claims, wherein the video tubelet comprises a video of a sub-volume of each of the plurality of ultrasound images.

13. An ultrasound imaging system comprising a processor configured to carry out the method of any one of claims 1 to 12.

14. The ultrasound imaging system of claim 13, further comprising a display configured to provide the determination of whether the tubelet comprises the pathology of interest.

15. A computer program product comprising instructions which when executed by a processor, cause the processor to carry out the method of any one of claims 1 to 12.
